# EUROPEAN PATENT SPECIFICATION

(11) **EP 0 429 245 B1**
(45) Date of publication and mention of the grant of the patent: **07.02.1996**
(21) Application number: 90312439.4
(22) Date of filing: 15.11.1990
(51) Int. Cl.: A61K 38/22, A61K 38/04, A61K 9/16, A61K 9/52

(54) **A therapeutic agent for central nervous diseases**
Therapeutisches Mittel für Erkrankungen des Zentralnervensystems
Agent thérapeutique pour les maladies du système central nerveux

(30) Priority: 17.11.1989 JP 300108/89
(43) Date of publication of application: 29.05.1991
(73) Proprietor: Takeda Chemical Industries, Ltd., Chuo-ku, Osaka (JP)
(72) Inventor: Nagaoka, Akinobu, Kawanishi, Hyogo 666-01 (JP); Ogawa, Yasuaki, Otokuni-gun, Kyoto 618 (JP); Fukuda, Naohisa, Kawanishi, Hyogo 666-01 (JP); Okada, Hiroaki, Suita, Osaka 565 (JP)
(74) Representative: Laredo, Jack Joseph

(56) References cited:
- EP-A- 0 134 582
- EP-A- 0 168 042
- EP-A- 0 256 726
- PATENT ABSTRACTS OF JAPAN, vol. 12, no. 103, (C-485)[2950], 5th April 1988; & JP-A-62 234 029
- JOURNAL MED. CHEM., vol. 27, no. 6, 1984, pages 741-745, American Chemical Society; T. SZIRTES et al.: "Synthesis of thyrotropin-releasing hormone analogues. 1. Complete dissociation of central nervous system effects from thyrotropin-releasing activity"
- INTERDISCIPL. TOPIES GERONT., vol. 19, 1985, pages 175-183, Karger, Basel, CH; C.M. YATES et al.: "Peptides and amines in Alzheimer-type dementia and Down's syndrome"
- PROC. WEST. PHARMACOL. SOC., vol. 30, 1987, pages 57-58; A. HORITA et al.: "Preliminary studies on the effects of a TRH analog, MK-771, in an animal model of Alzheimer's disease"

## Description

### Field of The Invention

The present invention relates to the preparation of a therapeutic composition for treatment of central nervous diseases.

### Background of the Invention

In recent years, the number of patients with senile dementia has been on the steady increase reflecting the increasing population in advanced age brackets and this is presenting very serious social problems. Much attention is being focused on the trend that (1) the patient population is ever increasing and that (2) because little is known about etiologic factors involved and, hence, no unerring drug or therapy can be indicated, the disease is virtually intractable.

Clinical manifestations of senile dementia are multiple, including not only disturbance of orientation to place, time or person and of memory but also decreased volition, emotional disturbance, depression and abnormal behavior. The therapeutic drugs commonly employed for senile dementia are psychotropic agents (mostly antianxiety and antidepressant drugs) and cerebral circulation improving agents, but none of them have proved as satisfactory as desired.

For the treatment of spinocerebellar degeneration, thyrotropin releasing hormone (TRH) is reported to be effective (Itsuro Sobue: Clinical Neurology 17:791∼799, 1977) but this substance must be administered daily and in large doses, the effect is not always satisfactory, and many side effects are inevitable.

Because of the vague etiologies of senile dementia and spirocerebellar degeneration, there is no unerring therapeutic drug or modality for these diseases and on the assumption that these are dysfunctions of the central nervous system, brain metabolism or neurotransmission modulators are generally indicated. However, these agents are not conducive to improvement in various symptoms, particularly memory disturbance. Generally, drugs are hard to pass the blood-brain barrier and must be administered in large doses to produce definable effects. In other words, the drugs tend to act on other tissues than the brain, too, thus causing side effects of necessity.

### Objects of the Invention

The main object of the present invention is to provide a pharmaceutical composition with the minimum potential to cause side effects and capable of producing clinically desirable effects in small doses in the treatment of senile dementia or spinocerebellar degeneration.

The inventors of the present invention explored into sustained-release pharmaceutical compositions adapted to release TRH or an analog thereof at controlled rates (cf. EP-A-0256726) and found that such sustained-release preparations, even in small doses, are able to markedly improve and cure symptoms of dementia in animal models and also activate the cerebral function. This finding and results of subsequent research have culminated in the accomplishment of the present invention.

### Summary of the Invention

According to the present invention, there is provided a pharmaceutical composition for the treatment of senile dementia or spinocerebellar degeneration which is adapted to release an oligopeptide having TRH activity sustainedly into the body and its use.

### Detailed Description of the Invention

As an example of said oligopeptide having TRH activity, there may be mentioned a compound of the formula 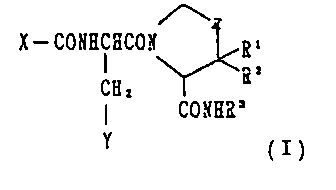
wherein X means a 4-, 5- or 6-membered heterocyclic group; Y means imidazol-4-yl, 4-hydroxyphenyl or isopropyl; R¹ and R² are the same or different and each means hydrogen or a lower alkyl group; R³ means hydrogen or an aralkyl group which may optionally be substituted; and Z means methylene. The 4-, 5- or 6-membered heterocyclic group may contain nitrogen, oxygen or/and sulfur atoms and includes, inter alia, 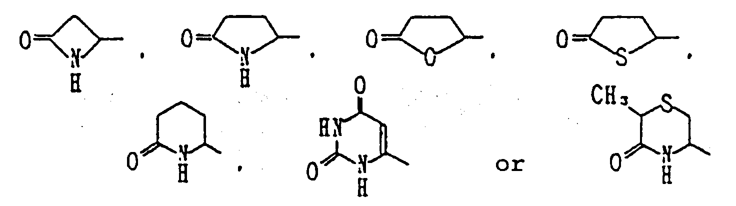
The lower alkyl group which is designated by R¹ and R² includes C₁₋₃ alkyl (e.g. methyl, ethyl, propyl and isopropyl).

The aralkyl group which may optionally be substituted, as designated by R³, includes, inter alia, phenyl C₁₋₃ alkyl such as 3,4-dihydroxyphenylethyl and so on.

Compound (I) preferably consists of 2 to 4 amino acids or derivatives or analogs thereof.

Among species of compound (I) are pyroGlu-His-ProNH₂(TRH), α-butyrolactonecarbonyl-His-ProNH₂(DN-1417), orotyl-His-ProNH₂(CG-3509), 2-methyltetrahydrothiazin-3-on-5-ylcarbonyl-His-ProNH₂(CG-3703), pyroGlu-Tyr-ProNH₂(Ro 102928), pyroGlu-His-ProNH(3,4-dihydroxyphenylethyl)(Ro 109430), pyroGlu-His-(3,4-dihydroxyphenylethylaminocarbonyl) (Ro 108802), pyro-2-aminoadipyl-His-thiazolidine-4-carbamide (Mk-771), pyro-2-aminoadipyl-Leu-ProNH₂(RGH-2202), pyroGlu-His-(3-monomethyl)ProNH₂(RX 74355), pyroGlu-His-(3,3-dimethyl)ProNH₂(RX 77368), azetidinon-4-ylcarbonyl-His-proNH₂(YM-14673) and so on. (For information on the properties of these compounds, the descriptions in Neuropharmacology 20, 947∼957, 1981 and 23, 339∼348, 1984, Brain Research Reviews 4, 389∼403, 1982, Brain Research 486, 228∼235, 1989, Arzneim Forsch./Drug Res. 39, 297∼298, 1989 and EP-A-123,444. Particularly preferred species of compound (I) are TRH, DN-1417 and CG-3509.

The above compound (I) can be used either in the free form or in the form of a pharmaceutically acceptable salt. Particularly preferred are the free compound and salts with weak acids having pKa values not less than 4.

The pharmaceutical composition of the invention, which contains said oligopeptide and is adapted to release it sustainedly may be supplied in the conventional sustained-release dosage forms for administration by suitable routes. It is usual to select a dosage form adapted to release the drug over a period not less than one week but insofar as the same effect can be achieved by more frequent administration, any dosage form suited to such therapeutic regimen can be employed. However, it is preferable that the preparation be able to release the drug over at least 24 hours and more desirable that it release the drug continuously for at least 2 days.

Typical dosage forms for releasing TRH or an analog thereof in a sustained manner are injections and pellets for implantation. A pellet prepared by dispersing the drug in a polymer matrix or an injection prepared by comminuting the same or processing the drug into microspheres or microcapsules are the most common examples. The polymer mentioned above is preferably biogradable or biosoluble and includes, inter alia, (1) synthetic polymers such as polylactic acid, polylacticglycolic acid, polycyanoacrylate, polycaprolactone, polyorthoester, polymalic acid, poly-β-hydroxybutyric acid, polyorthocarbonate, maleic anhydride copolymer, etc., (2) proteins such as albumin, polyamino acid, collagen, gelatin, etc., and (3) carbohydrates such as polyacrylstarch, degradable starch, dextran and its derivatives, methylcellulose, ethylcellulose, hydroxypropylcellulose, acetylcellulose, nitrocellulose and so on.

These sustained-release preparations can be manufactured by the established pharmaceutical procedures. The drug is preferably dispersed or dissolved evenly in the polymer matrix. In this connection, the applicable manufacturing process may comprise dissolving the drug and the matrix polymer evenly in an appropriate solvent and removing the solvent to provide a pellet or alternatively mixing or emulsifying an aqueous solution of the drug and a non-aqueous solution of the polymer evenly and removing the solvent and water to provide a pellet. Such a pellet can be pulverized to give an injection. The techniques available for the manufacture of microspheres include the phase separation technique, the drying-in-liquid technique (solvent removal method), and coacervation technique. Among other types of controlled release preparations are transdermal therapeutic systems. These may be patches or ointments, although patches are more advantageous for long-term sustained release. Transdermal therapeutic systems can be manufactured by the reported processes. The bases to be used should be materials in which TRH or any other TRH-active substance can be readily dissolved or dispersed, such as various polyalcohols, e.g. propylene glycol, sorbitol solution, glycerin, polyethylene glycol, etc., vegetable oils (e.g. olive oil), animal oils (e.g. squalene, lanolin, etc.), mineral oils (e.g. liquid paraffin, vaseline, etc.), and other oleochemicals inclusive of fatty acid esters. Various percutaneous absorption promoting agents may be used in conjunction. Among such agents are fatty alcohols, fatty acid alkali metal salts, aliphatic monoamines, Azone and so on.

The dosage varies with the degree of activity of the drug. Generally when the dosage form is a one-week controlled release preparation, for instance, sufficient efficacy can be expected with a single dose of 0.35 to 35 mg (0.007 ∼ 0.7 mg/kg body weight). As will be apparent from the experiments described hereinafter, such a sustained-release dosage form not only helps reduce the necessary dosage as compared with the daily administration of an aqueous solution but also insures the efficacy not attainable by the latter dosage form. Thus, sufficient efficacy is assured even when the dosage is 0.05 to 5 mg (0.001 to 0.1 mg/kg body weight). Generally in the daily administration of aqueous TRH, 1 to 4 mg is administered twice a day, which corresponds to a weekly dosage of 14 to 56 mg (0.28 ∼ 1.12 mg/kg body weight). At this dose level, however, many side effects appear and yet the effect is not always satisfactory. [Naohiko Takahitoda et al.: "Shinkei Peptaido-no-Kiso-to-Rinsho" The Fundemental and Clinical Aspects of Neuropeptides (ed. by Itsuro Sobue), Ministry of Health and Welfare New Drug Development Research Publication, 266 ∼ 272, 1986; Itsuro Sobue: Neurological Therapeutics 3(4), 303 ∼ 309, 1986]. It seems logical to presume that a reduced dosage, as it is true with the present invention, leads to a reduced risk for side effects.

The concentration of the drug in the pharmaceutical composition is not critical and provided that the drug may be released continuously over a prolonged time period, the concentration is preferably as high as possible. Generally a slow release preparation tends to release more of the drug it contains in an early phase (0.5 ∼ 24 hours) after administration than in the subsequent period. (This early massive release of the drug is known as an initial burst). If this initial burst is large, the drug may induce adverse reactions. Therefore, the amount of release up to 6 hours after administration is preferably controlled within 3 mg. If follows, then, that the drug concentration of the preparation is preferably in the range of 0.1 to 50, preferably 1 to 20 percent (w/w).

In the practice of the present invention, the drug is processed into microspheres with a homopolymer or copolymer of lactic acid and/or glycolic acid and the microspheres are suspended in water for subcutaneous administration. By this procedure, excellent drug activity can be insured.

The following examples and experiment examples are merely intended to illustrate the invention in further detail and should by no means be construed as defining the metes and bounds of the invention.

### Experiment Example 1

Mice in which amnesia was established by administration of cycloheximide beforehand were dosed with the microencapsulated TRH of Example 2 and an aqueous solution of TRH tartarate, respectively, to compare the therapeutic effects.

### Method

Using male mice (C57BL strain, 5-week-old), a memory disturbance model was constructed by subcutaneous administration of the protein synthesis inhibitor cycloheximide (60 mg/kg). The memory disturbance was assessed by the passive avoidance test. Thus, using a test apparatus comprising a light compartment and a dark compartment, the mouse was first put in the light compartment and taking advantage of the behavior of mice liking dark quarters, the door between the two compartments was closed upon entry of the mouse into the dark compartment and an electric shock was applied from the floor (acquisition session). The administration of cycloheximide was carried out 30 minutes before this acquisition trial. On the following day, the mouse was put in the light compartment again and the time to entry of the mouse into the dark compartment was measured (test session). The normal mice remembered the electric shock they had experienced on the previous day but the mice given cycloheximide could not recall the experience and moved into the dark compartment with a short latency time. In the above memory disturbance model, the microencapsulated TRH of Example 2 was subcutaneously administered in a single dose of 0.008, 0.04 or 0.2 mg (as TRH)/kg/day as TRH 9 days preceding the test session. For control purposes, an aqueous solution of TRH tartarate was administered in a dose of 0.2 mg/kg/day once a day for 9 consecutive days. In the groups including this latter group, the passive avoidance learning session was conducted.

### Results

As apparent from Table 1, whereas the latency in normal mice in the test session was 300 seconds (observation time: 5 minutes), the latency in the cycloheximide groups was as short as 17.4 seconds on the average. In this amnesia model, the administration of the microencapsulated slow-release TRH produced dose-related improvements, prolonging the latency accordingly. The differences were statistically significant at 0.04 mg/kg/day and higher doses. On the other hand, the daily repeated subcutaneous administration of the aqueous solution of TRH tartarate failed to prolong the latency at all despite the multiple dosing of 0.2 mg (as TRH)/kg/day.

It was, therefore, clear that the sustained-release TRH exhibits efficacy at a very low dose in this amnesia model.

**Table 1**

| Efficacy of the sustained-release TRH in cycloheximide-induced amnesia | |
|---|---|
| Animal Group | Latency (seconds) |
| Normal control | 300 |
| Amnesia | 17.4 |
| Amnesia + microencapsulated TRH (0.008 mg/kg/day as TRH) | 17.9 |
| Amnesia + microencapsulated TRH (0.04 mg/kg/day as TRH) | 27.7* |
| Amnesia + microencapsulated TRH (0.2 mg/kg/day as TRH) | 53.2* |
| Amnesia + aqueous solution of TRH (0.2 mg/kg/day as TRH) | 18.0 |
| Number of animals: 10 per group | |

| | |
|---|---|
| *: p<0.01 (compared with amnesia group) | |

### Experiment 2

Pentobarbital sleeping time-shortening effect (brain function stimulating effect)

### Method

The microencapsulated sustained-release TRH of Example 2 was used as the test material.
(1) Administration of microencapsulated TRH. The microencapsulated TRH of Example 2 was dispersed in aqueous medium and the suspension was injected subcutaneously at the back of male rats (JCL:Wistar, 7-week-old) at a dose of 0.05, 0.2 or 0.5 mg/kg/day. On days 4, 11, 18 and 25 after administration, 30 mg/kg of pentobarbital was injected into the caudal vein and the time of loss of righting reflex was measured as sleeping time. The control group received the dispersion medium.
(2) Administration of aqueous TRH tartarate solution. An aqueous solution of TRH tartarate was subcutaneously administered to rats in doses of 3, 10, 20 and 50 mg as TRH/kg in otherwise the same manner as above and the time of loss of righting reflex was measured. The control group received physiological saline.

### Results

As apparent from Tables 2 and 3, administration of the microencapsulated sustained-release TRH significantly shortened the sleeping time on days 4 and 11 after administration at dose levels of 0.05 and 0.2 mg/kg/day and on days 4, 11 and 18 at 0.5 mg/kg/day. These effects were no longer found on day 25 when the drug had ceased to be released. On the other hand, administration of the aqueous solution of TRH tartarate shortened the sleep time dose-dependently, with significant effects found at 10 mg/kg and higher doses. Thus, the microencapsulated sustained-release TRH shortened pentobarbital-induced sleeping time and the minimum effective dose was about 1/200 -fold that of the aqueous solution.

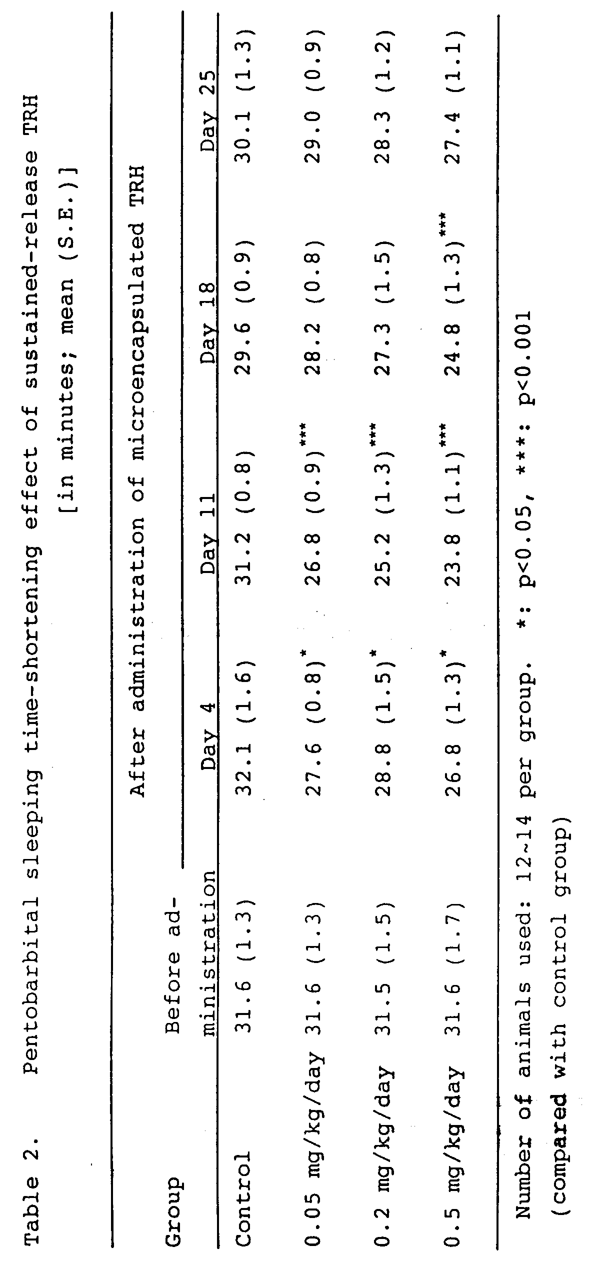

**Table 3**

| Pentobarbital sleeping time-shortening effect of aqueous TRH tartarate solution [in minutes; mean (S.E.)] | |
|---|---|
| Group | Sleep time |
| Control | 69.0 (2.6) |
| 3 mg/kg | 70.6 (4.2) |
| 10 mg/kg | 58.6 (2.5)* |
| 20 mg/kg | 56.0 (4.0)* |
| 50 mg/kg | 45.6 (1.6)*** |
| Number of test animals: 5∼10 per group | |

| | |
|---|---|
| *: p<0.05, | |
| ***: p<0.001 (compared with control group) | |

### Experiment Example 3

Effect on the low-anxiety-like behavior (decreased alertness) of senility-accelerated mice (SAM) in the plus maze test

### Method

The effect of TRH on the exploratory behavior of 8-month-old male SAM-R/1 (normal control mice) and SAM-P/8 (senility-accelerated mice) in the plus maze by the single subcutaneous administration of the microencapsulated sustained-release TRH of Example 2 as suspended in aqueous dispersion medium or the repeated subcutaneous administration of an aqueous solution of TRH tartarate and the effects of the two drugs were compared. The experiment was performed in two independent trials, one with the microencapsulated TRH and the other with the aqueous solution of TRH tartarate, using different mice in groups of 10 each.

The microencapsulated TRH was subcutaneously administered in the dorsal cervical region at a dose of 0.05 or 0.2 mg/kg/day. The SAM-R/1 control and SAM-R/8 control groups received the dispersion medium. On the other hand, the aqueous solution of TRH tartarate was subcutaneously administered in the dorsal cervical region at a dose of 0.05 or 0.2 mg/kg between 10 and 11 a.m. every day. The control group received physiological saline. The plus maze experiment was performed 30 minutes after administration of the microencapsulated TRH or after administration of the aqueous solution of TRH tartarate on day 6 after the beginning of the repeated administration.

The overhead plus maze apparatus was a cruciform structure having four horizontal arms (10 x 50 cm) at the height of 50 cm above floor level, with the two opposed arms being walled arms each surrounded by 50 cm-high walls on the three sides except the side adjoining the central platform and the other two arms mouse was placed on the central platform, its exploratory behavior was observed for 5 minutes and the number of entries into either walled arm was counted.

### Results

As apparent from Table 4, the SAM-P/8 control groups given the dispersion medium or physiological saline showed significant increases in the open arm entry rate compared with the SAM-R/1 control group. This increase in the entry rate was significantly and markedly inhibited by administration of 0.05 or 0.2 mg/kg/day of the microencapsulated sustained-release TRH. On the other hand, repeated administration of the aqueous solution of TRH tartarate produced no significant effect but showed a tendency of slight inhibition only in the high dose group. The increase in the open arm entry rate in the SAM-P/8 group was apparently attributable to decreased anxiety (lowered level of alertness) and it was found that this low anxiety-like behavior was markedly releaved by administration of the slow-release TRH.

**Table 4**

| Effect on then exploratory behavior of senility-accelerated mice (SAM) in the plus maze test | | | |
|---|---|---|---|
| Animal | Drug | Dosage | % Open arm entry rate [mean (standard error)] |
| SAM-R/1 | Dispersion medium | - | 22.2 (3.6) |
| SAM-P/8 | Dispersion medium | - | 40.0 (1.2)** |
| SAM-P/8 | Microencapsulated TRH | 0.05 mg/kg/day | 28.2 (3.0)⁺⁺ |
| SAM-P/8 | Microencapsulated TRH | 0.2 mg/kg/day | 29.3 (3.4)⁺⁺ |
| SAM-R/1 | Saline | - | 20.6 (2.8) |
| SAM-P/8 | Saline | - | 33.6 (3.1)** |
| SAM-P/8 | Aqueous TRH solution | 0.05 mg/kg/day | 32.7 (2.9)** |
| SAM-P/8 | Aqueous TRH solution | 0.2 mg/kg/day | 29.2 (3.2)* |
| Number of animals: 10 per group Saline = physiological saline | | | |

| | | | |
|---|---|---|---|
| *: p<0.05; | | | |
| **: p<0.01 (compared with SAM-R/1 control) | | | |
| ++: p<0.01 (compared with SAM-P/8 control) | | | |

### Experiment Example 4

Effect on the rolling mouse Nagoya (hereditary ataxia model)

### Method

This experiment was performed in rolling mice Nagoya (10 males and 5 females, weighing 15.5∼34.0 g). The microencapsulated TRH of Example 2 was dissolved in aqueous dispersion medium and the suspension was administered subcutaneously at a dose of 0.05 or 0.2 mg/kg/day. On days 2, 9 and 16 after administration, the mouse was placed on a metallic drum measuring 10 cm in height and 5 cm in diameter and the duration of stay of the animal on the drum was measured 4 times at 3-minute intervals. The average of results was calculated. The control group received the dispersion medium only. As a positive control, an aqueous solution of TRH tartarate was administered in a single subcutaneous dose of 3 or 10 mg as TRH/kg and starting minutes after administration, the stay time was measured 4 times at 3-minute intervals. The results were averaged.

### Results

As apparent from Table 5, the microencapsulated TRH caused a significant prolongation of the stay time over the control group on day 2 after administration at 0.05 mg/kg/day and on days 2 and 9 after administration at 0.2 mg/kg/day. These effects were no longer observed on day 16 when the drug had ceased to be released. The results of administration of the aqueous solution of TRH tartarate are shown in Table 6. At the two dose levels used, the aqueous solution caused a significant prolongation of stay time but required an approximately 60-fold dose compared with the microencapsulated TRH to achieve comparable effects.

**Table 5**

| Effect of sustained-release TRH on rolling mice Nagoya (hereditary ataxia model) | | | | | |
|---|---|---|---|---|---|
| Drug | Dosage | Stay time [in seconds; mean (S.E.)] | | | |
| | | Day 3 before administration | Day 2 after administration | Day 9 after administration | Day 16 after administration |
| Dispersion medium (control) | - | 36.5(5.6) | 24.2(3.4) | 32.3(4.2) | 33.9(5.3) |
| Microencapsulated TRH | 0.05 mg/kg/day | 32.2(5.6) | 43.0(7.0)* | 44.7(7.8) | 42.2(7.9) |
| Microencapsulated TRH | 0.2 mg/kg/day | 38.3(8.6) | 41.5(6.0)* | 46.5(6.0)* | 40.1(4.8) |
| Number of test animals: 15 per group | | | | | |

| | | | | | |
|---|---|---|---|---|---|
| *: p<0.05 | | | | | |

**Table 6**

| Effect of aqueous TRH tartarate solution on rolling mice Nagoya (hereditary ataxia model) | | |
|---|---|---|
| Drug | Dosage | Stay time [in seconds; mean (S.E.)] |
| Dispersion medium (control) | - | 12.9 (1.6) |
| Aqueous TRH | 3 mg/kg | 20.3 (2.5)* |
| Aqueous TRH | 10 mg/kg | 25.3 (4.2)** |
| Number of test animals: 15 per group | | |

| | | |
|---|---|---|
| *: p<0.05; | | |
| **: p<0.01 | | |

### Example 1

In 0.625 ml of water was dissolved 250 mg of TRH (solution A). In 6.25 ml of methylene chloride was dissolved a polylactic-glycolic acid (lactic acid/glycolic acid = 75/25, average molecular weight 14,000; hereinafter referred to as PLGA (75/25)-14000) (solution B). Using a compact homogenizer (Polytron, Finematica, Switzerland), solution A was added to solution B with stirring to give a W/O emulsion. After cooling this emulsion to 18°C, it was poured in 1,250 ml of a 0.25% aqueous solution of polyvinyl alcohol (PVA) and worked up in a turbine homomixer to give a W/O/W emulsion. Then, while this W/O/W emulsion is gently stirred, the methylene chloride was evaporated off to solidify the internal W/O/W emulsion and the solid matter was collected by means of a centrifuge to give microcapsules in which TRH had been entrapped (the entrapped ratio: 100%<). The microcapsules were then freeze-dried for further removal of the solvent and water to provide a powder. The controlled TRH releasability of the microcapsules in vitro and in vivo following subcutaneous administration to rats was investigated by determining the residual amount of TRH in the release medium or at the site of administration). The results are shown in Table 7. In both the in vitro and in vivo experimental systems, TRH was released from the microcapsules continuously over a period of 4 weeks.

**Table 7**

| In vitro and in vivo release patterns (% Residue relative to initial concentration), n=3 | | | | | |
|---|---|---|---|---|---|
| | Day 1 | Week 1 | Week 2 | Week 3 | Week 4 |
| in vitro | 94.5 | 82.2 | 59.0 | 44.6 | 7.8 |
| in vivo | 79.8 | 72.8 | 31.3 | 16.7 | 3.2 |

### Example 2

A two-week controlled release microencapsulated TRH was prepared using PLGA (75/25)-10,000 in lieu of PLGA (75/25)-14,000 in otherwise the same manner as Example 1.
The entrapped ratio was 100%<.

### Example 3

A 4-week controlled release microencapsulated CG-3509 was prepared using CG-3509 in lieu of TRH in otherwise the same manner as Example 1. The entrapped ratio was 94.5%.

### Example 4

In 1 ml of dioxane was dissolved 50 mg of PLGA (75/25)-10,000 followed by addition of 50 µl of an aqueous solution containing 10 mg of TRH to prepare a mixed suspension. This suspension was cast in a tray and the solvent was evaporated off under a nitrogen blanket. The resulting film was dried in vacuo for complete removal of the residual solvent and water (the entrapped ratio was 100%). The procedure gave a therapeutic film adapted to release TRH sustainedly over a period of 2 weeks.

### Example 5

The film prepared in Example 4 was finely divided and sieved through a 170-mesh sieve to give a powdery preparation adapted to release TRH over a period of 2 weeks.

### Example 6

In a mixture of 170 mg of propylene glycol and 30 mg of oleic acid was dissolved 1.0 mg of TRH tartarate and the solution was absorbed into a 2 mm-thick porous ceramic substrate. This was covered with aluminum foil on one side to give a therapeutic preparation for sustained release of TRH into the body.

## Claims

1. Use of an oligopeptide having TRH activity in the preparation of a sustained-release pharmaceutical composition for the treatment of senile dementia or spinocerebellar degeneration, in a dosage from selected from injections, pellets for implantation and transdermal therapeutic systems, wherein said oligopeptide being continuously releasable from said sustained release pharmaceutical composition over a period of time of at least 24 hours both in vitro and in vivo.

2. Use according to Claim 1, wherein said oligopeptide is a compound of the formula: 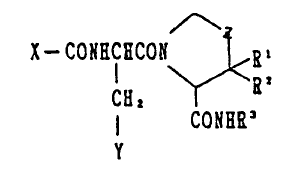 wherein X is a 4, 5- or 6-membered heterocyclic group, Y is imidazol-4-yl or 4-hydroxyphenyl, R¹ and R² are the same or different and each is hydrogen or lower alkyl, R³ is hydrogen or optionally substituted aralkyl and Z is methylene, or a pharmaceutically acceptable salt thereof.

3. Use according to Claim 2, wherein X is a moiety selected from:

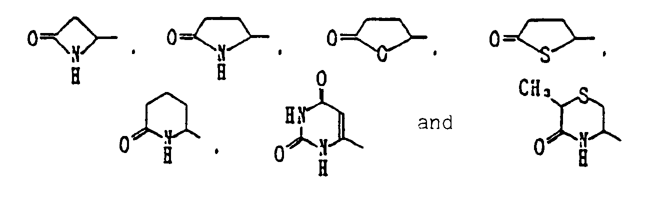

4. Use according to Claim 2 or Claim 3, wherein R¹ and R² are each hydrogen.

5. Use according to any of Claims 2 to 4, wherein R³ is hydrogen or 3,4-dihydroxyphenylethyl.

6. Use according to Claim 2, wherein said oligopeptide is a compound selected from pyroGlu-His-ProNH₂, α-butyrolactonecarbonyl-His-ProNU₂ and orotyl-His-ProNH₂.

7. Use according to any of Claims 1 to 6, wherein said oligopeptide is in the free form.

8. Use according to any of Claims 1 to 6, wherein said oligopeptide is in the form of a pharmaceutically acceptable salt with an inorganic acid or an organic acid.

9. Use according to any of Claims 1 to 8, wherein the concentration of said oligopeptide is in the range of from 0.1 to 50 percent (w/w).

10. Use according to any of Claims 1 to 9, wherein said sustained release pharmaceutical composition is in the form of an injection which is prepared by comminuting a pellet prepared by dispersing said oligopeptide in a polymer matrix, or by processing said oligopeptide into microspheres or microcapsules.

11. Use according to any of Claims 1 to 9, wherein said sustained release pharmaceutical composition is in the form of implanation pellets which are prepared by dispersing said oligopeptide in a polymer matrix.

12. Use according to any of claims 1 to 11, wherein said oligopeptide is present in a dosage of 0.001 to 0.1 mg/kg body weight.

13. Use according to any of Claims 1 to 12, wherein said sustained-release pharmaceutical composition is for the treatment of spinocerebellar degeneration.

## Patentansprüche

1. Verwendung eines Oligopeptids mit TRH-Aktivität für die Herstellung einer pharmazeutischen Zusammensetzung mit verzögerter Freisetzung für die Behandlung der senilen Demenz oder der spinozerebellären Degeneration in einer Dosierungsform, ausgewählt aus Injektionen, Pellets für die Implantation und transdermalen therapeutischen Systemen, wobei das Oligopeptid kontinuierlich aus der pharmazeutischen Zusammensetzung mit verzögerter Freisetzung über einen Zeitraum von wenigstens 24 Stunden sowohl in vitro als auch in vivo freisetzbar ist.

2. Verwendung nach Anspruch 1, wobei das Oligopeptid eine Verbindung der Formel: 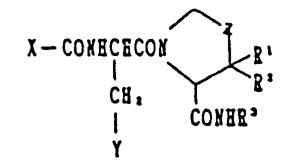 ist, worin X eine 4-, 5- oder 6-gliedrige heterocyclische Gruppe ist, Y Imidazol-4-yl oder 4-Hydroxyphenyl ist, R¹ und R² dieselben oder unterschiedliche sind und jedes Wasserstoff oder ein Niederalkyl ist, R³ Wasserstoff oder ein wahlweise substituiertes Aralkyl ist und Z Methylen ist, oder ein pharmazeutisch annehmbares Salz davon.

3. Verwendung nach Anspruch 2, wobei X ein Rest ist, ausgewählt aus:

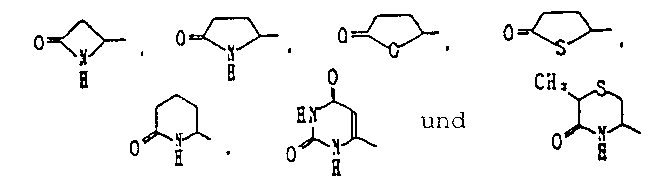

4. Verwendung nach Anspruch 2 oder Anspruch 3, wobei R¹ und R² jeweils Wasserstoff sind.

5. Verwendung nach einem der Ansprüche 2 bis 4, wobei R³ Wasserstoff oder 3,4-Dihydroxyphenylethyl ist.

6. Verwendung nach Anspruch 2, wobei das Oligopeptid eine aus pyroGlu-His-ProNH₂, α-Butyrolactoncarbonyl-His-ProNH₂ und Orotyl-His-ProNH₂ ausgewählte Verbindung ist.

7. Verwendung nach einem der Ansprüche 1 bis 6, wobei das Oligopeptid in der freien Form vorliegt.

8. Verwendung nach einem der Ansprüche 1 bis 6, wobei das Oligopeptid in Form eines pharmazeutisch annehmbaren Salzes mit einer anorganischen Säure oder einer organischen Säure vorliegt.

9. Verwendung nach einem der Ansprüche 1 bis 8, wobei die Konzentration des Oligopeptids im Bereich von 0,1 bis 50 Gew.-% liegt.

10. Verwendung nach einem der Ansprüche 1 bis 10, wobei die pharmazeutische Zusammensetzung mit verzögerter Freisetzung in Form einer Injektion vorliegt, die durch das Pulverisieren eines durch Dispergieren des Oligopeptids in eine Polymermatrix hergestellten Pellets oder durch das Verarbeiten des Oligopeptids in Mikrokugeln oder Mikrokapseln hergestellt wird.

11. Verwendung nach einem der Ansprüche 1 bis 9, wobei die pharmazeutische Zusammensetzung mit verzögerter Freisetzung in Form von Implantations-Pellets vorliegt, die durch das Dispergieren des Oligopeptids in einer Polymermatrix hergestellt werden.

12. Verwendung nach einem der Ansprüche 1 bis 11, wobei das Oligopeptid in einer Dosis von 0,001 bis 0,1 mg/kg Körpergewicht vorhanden ist.

13. Verwendung nach einem der Ansprüche 1 bis 12, wobei die pharmazeutische Zusammensetzung mit verzögerter Freisetzung zur Behandlung der spinozerebellären Degeneration dient.

## Revendications

1. Utilisation d'un oligopeptide possédant une activité de thyrolibérine (TRH) dans la préparation d'une composition pharmaceutique à libération prolongée, destinée au traitement de la démence sénile ou de la dégénérescence cérébrospinale, sous une forme galénique choisie parmi les solutions injectables, les pastilles à implanter et les systèmes thérapeutiques transdermiques, ledit oligopeptide pouvant être continuellement libéré par ladite composition pharmaceutique à libération prolongée pendant un laps de temps d'au moins 24 heures, tant in vitro qu'in vivo.

2. Utilisation conforme à la revendication 1, dans laquelle ledit oligopeptide est un composé de formule : 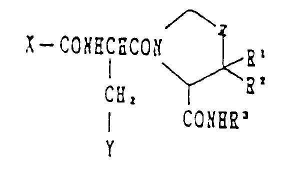 dans laquelle X représente un groupe hétérocyclique à 4, 5 ou 6 chaînons, Y représente un groupe imidazol-4-yle ou 4-hydroxyphényle, R¹ et R² sont identiques ou différents et représentent chacun un atome d'hydrogène ou un groupe alkyle inférieur, R³ représente un atome d'hydrogène ou un groupe aralkyle portant éventuellement des substituants, et Z représente un groupe méthylène,
ou un sel d'un tel composé, acceptable en pharmacie.

3. Utilisation conforme à la revendication 2, dans laquelle X représente un groupe choisi parmi :

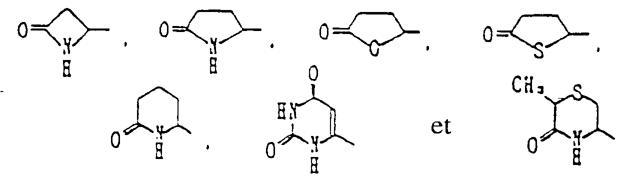

4. Utilisation conforme à la revendication 2 ou 3, dans laquelle R¹ et R² représentent chacun un atome d'hydrogène.

5. Utilisation conforme à l'une des revendications 2 à 4, dans laquelle R³ représente un atome d'hydrogène ou un groupe 3,4-dihydroxyphényl-éthyle.

6. Utilisation conforme à la revendication 2, dans laquelle ledit oligopeptide est un composé choisi parmi : pyroGlu-His-ProNH₂, α-butyrolactonecarbonyl-His-ProNH₂, et orotyl-His-ProNH₂.

7. Utilisation conforme à l'une des revendications 1 à 6, dans laquelle ledit oligopeptide se trouve sous sa forme libre.

8. Utilisation conforme à l'une des revendications 1 à 6, dans laquelle ledit oligopeptide se trouve sous la forme d'un sel, acceptable en pharmacie, d'un acide minéral ou organique.

9. Utilisation conforme à l'une des revendications 1 à 8, dans laquelle la concentration dudit oligopeptide se situe dans l'intervalle allant de 0,1 à 50 % p/p.

10. Utilisation conforme à l'une des revendications 1 à 9, dans laquelle ladite composition pharmaceutique à libération prolongée se trouve sous la forme d'une solution injectable que l'on a préparée en pulvérisant une pastille préparée par dispersion dudit oligopeptide dans une matrice polymère, ou en mettant ledit oligopeptide en microsphères ou dans des microcapsules.

11. Utilisation conforme à l'une des revendications 1 à 9, dans laquelle ladite composition pharmaceutique à libération prolongée se trouve sous la forme de pastilles à implanter, préparées par dispersion dudit oligopeptide dans une matrice polymère.

12. Utilisation conforme à l'une des revendications 1 à 11, dans laquelle ledit oligopeptide se trouve en une dose de 0,001 à 0,1 mg/kg de poids corporel.

13. Utilisation conforme à l'une des revendications 1 à 12, dans laquelle ladite composition pharmaceutique à libération prolongée est destinée au traitement de la dégénérescence cérébrospinale.
